# FASCICULE DE BREVET EUROPEEN

(11) **EP 0 783 578 B1**
(45) Date de publication et mention de la délivrance du brevet: **06.06.2007**
(21) Numéro de dépôt: 95932048.2
(22) Date de dépôt: 22.09.1995
(51) Int. Cl.: C12N 15/31, C12N 1/19, C12Q 1/02, C12R 1/85

(54) **LEVURE A PERMEABILITE MODIFIEE**
HEFE MIT GEANDERTER PERMEABILITÄT
YEAST WITH MODIFIED PERMEABILITY

(30) Priorité: 27.09.1994 FR 9411509
(43) Date de publication de la demande: 16.07.1997
(73) Titulaire: Aventis Pharma S.A., 92160 Antony (FR)
(72) Inventeur: FLEER, Reinhard, F-91440 Bures-sur-Yvette (FR); MARCIREAU, Christophe, F-75013 Paris (FR); SCHERMAN, Daniel, F-75645 Paris cédex 13 (FR)
(74) Mandataire: Le Coupanec, Pascale A.M.P.
(86) Numéro de dépôt international: PCT/FR1995/001226
(87) Numéro de publication internationale: WO 1996/010082

(56) Documents cités:
- J. BIOL. CHEM. (1993), 268(8), 5345-8 CODEN: JBCHA3;ISSN: 0021-9258, SHAH, NICKY ET AL 'Brefeldin A reversibly inhibits secretion in Saccharomyces cerevisiae'
- J. BIOL. CHEM. (1993), 268(5), 3040-3 CODEN: JBCHA3;ISSN: 0021-9258, VOGEL, JOSEPH P. ET AL 'Brefeldin A causes a defect in secretion in Saccharomyces cerevisiae'
- CHEMICAL ABSTRACTS, vol. 91, no. 5, 30 Juillet 1979 Columbus, Ohio, US; abstract no. 35411, LEES, NORMAN D. ET AL 'ESR determination of membrane order parameter in yeast sterol mutants' & BIOCHIM. BIOPHYS. ACTA (1979), 553(3), 469-75 CODEN: BBACAQ;ISSN: 0006-3002,
- CHEMICAL ABSTRACTS, vol. 90, no. 11, 12 Mars 1979 Columbus, Ohio, US; abstract no. 83505, KLEINHANS, F. W. ET AL 'ESR determinations of membrane permeability in a yeast sterol mutant' & CHEM. PHYS. LIPIDS (1979), 23(2), 143-54 CODEN: CPLIA4;ISSN: 0009-3084,
- MOL. GEN. GENET. (1993), 236(2-3), 214-18 CODEN: MGGEAE;ISSN: 0026-8925, SERVOS, JORG ET AL 'Gene SNQ2 of Saccharomyces cerevisiae, which confers resistance to 4-nitroquinoline-N-oxide and other chemicals, encodes a 169 kDa protein homologous to ATP-dependent permeases' cité dans la demande
- GENETICS (1994), 136(2), 505-15 CODEN: GENTAE;ISSN: 0016-6731, DEXTER, DWAYNE ET AL 'mutations in the yeast PDR3, PDR4, PDR7 and PDR9 pleiotropic (multiple) drug resistance loci affect the transcript level of an ATP binding cassette transporter encoding gene, PDR5'
- YEAST, vol. 10, pages 851-869, F. KLIS 'Review: Cell wall assembly in yeast' cité dans la demande
- PROCEEDINGS OF THE NATIONAL ACADEMY OF SCIENCES OF USA, vol. 90, WASHINGTON US, pages 2281-2285, M. HARA ET AL 'Identification of Ras farnesyltransferase inhibitors by microbial screening' cité dans la demande

### LISTE DE SEQUENCES

(1) INFORMATIONS GENERALES:
   (i) DEPOSANT:
      (A) NOM: RHONE POULENC RORER S.A.
      (B) RUE: 20, Avenue Raymond Aron
      (C) VILLE: ANTONY
      (E) PAYS: FRANCE
      (F) CODE POSTAL: 92165
      (G) TELEPHONE: 40.91.69.22
      (H) TELECOPIE: (1) 40.91.72.96
   (ii) TITRE DE L' INVENTION: LEVURE A PERMEABILITE MODIFIEE
   (iii) NOMBRE DE SEQUENCES: 2
   (iv) FORME DECHIFFRABLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 1:
      TTACTTTCGA TTTAAGTTTT ACATAATTTA AAA
(2) INFORMATIONS POUR LA SEQ ID NO: 2:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 2:
      GGCCTGCTCA TGAACGTGCT ATCTT

## Description

La présente invention concerne des levures modifiées possédant une perméabilité cellulaire non conforme à celle d'origine, induite par mutation génétique.

La levure est un modèle de cellules eucaryotes et un outil de choix pour étudier les voies métaboliques, le cycle cellulaire, leurs systèmes de régulation respectifs, l'isolement de gènes, la production de protéines hétérologues, etc... La levure peut également être utilisée pour la biotransformation de molécules d'intérêt ou pour la découverte de composés biologiquement actifs par criblage *in vivo.* Ces dernières applications nécessitent la pénétration des xénobiotiques à l'intérieur des cellules de levure.

La pénétration de ces molécules, de même que leur concentration, à l'intérieur des cellules de levure, dépend de différents paramètres régissant la "perméabilité" cellulaire. Ces différents paramètres interviennent principalement à trois niveaux distincts chez la levure, sa paroi cellulaire, sa membrane plasmique et ses mécanismes de détoxification.

Ainsi, pour qu'une molécule puisse pénétrer à l'intérieur des cellules de levure et y être biotransformée ou y exercer son effet biologique dans le cas d'un criblage, il lui faut franchir la paroi (composée essentiellement de glucanes, de mannoprotéines et de chitines) et la membrane plasmique (composée de stérols, de phospholipides et de protéines), deux barrières biologiques qui peuvent s'opposer faiblement ou fortement à sa pénétration. Pour maintenir une concentration élevée à l'intérieur de la cellule, elle ne doit pas être détoxifiée, par exemple par réexpulsion vers le milieu externe ou par métabolisation au sein de la cellule.

La littérature a rapporté tout une série de gènes impliqués dans la biosynthèse, la constitution et/ou l'intégrité de la paroi, de la membrane et au niveau des systèmes de détoxification.

Différents gènes interviennent dans la synthèse de la paroi comme la série des gènes *KRE* (Hutchins, 1983; Boone, 1990; Meader, 1990; Hill, 1992; Roemer, 1991; Brown,1993; Klis, 1994), *KTR* (Lussier, 1993; Klis, 1994) et *MNN* (Kanik, 1990; Klis, 1994; Ballou, 1982). Différents mutants de paroi ont pu être isolés comme les mutants *csd, chs, och, skn1, pmr1, pmt1, erd1, vgr1, vgr4, cal3, shc1, pkc1, bck1, mkk1, mkk2, mpk1, ppz1, ppz2, pmi1* (Klis, 1994). Les gènes *DHS1* (Lee, 1994), *SRB1* (Stateva, 1991), ainsi que les gènes impliqués dans la voie de signalisation de la protéine kinase C qui contrôle négativement la dégradation de la paroi par différentes glucanases comme *BGL2* (Shimizu, 1994; Klis, 1994), sont plus particulièrement concernés par l'intégrité de cette paroi.

Il a également été identifié tout un ensemble de gènes impliqués dans la biosynthèse de l'ergostérol qui est une forme majoritaire des stérols de champignons. Les stérols sont des constituants membranaires. A titre représentatif de ces gènes, on peut plus particulièrement citer les gènes *ERG10* (Dequin, 1988), *ERG11* et ERG13 (Servouse, 1984), *HMG1* et *HMG2* (Basson, 1986), *ERG12* (Oulmouden, 1988), *ERG8* (Tsay, 1991), *ERG19* (Mons, 1984), *IDI1* et *ERG20* (Anderson, 1989), *ERG9* (Fegueur, 1991), *ERG1* (Hoegenauer, 1991), *ERG18* (Karst, 1977), *ERG7* et *ERG17* (Karst, 1977), *ERG16* (Kalb, 1987), ERG24 (Marcireau, 1992), *ERG6* (Hardwick, 1994), *ERG2* (Ashman, 1991), *ERG3* (Arthington, 1991), *ERG5* (Molzhan, 1972), *ERG4* (Lai, 1994). Les Phospholipides sont également des constituants des membranes. Différents gènes impliqués dans la synthèse des phospholipides ont pu être identifiés comme *CHO1* (Kiyono, 1987), *CHO2* (Kodaki, 1991), *PEM2* (Kodaki, 1987), *CKI1* (Hosaka, 1989), *CCT1* (Tsukagoshi, 1987), *EPT1* et *CPT1* (Hjelmstad, 1991), *INO1* (Dean-Johnson, 1989), *PIS1* (Nikawa, 1987) etc. De même, différents gènes impliqués dans la synthèse des acides gras ont pu être mis en évidence. L'acétyl-CoA carboxylase (gène ACC1 (Al-Fell, 1992)) permet de produire du malonyl CoA qui sera utilisé par la synthase d'acides gras (gènes *FAS1* (Schwezer, 1986) et *FAS2* (Mohamed, 1990)) pour donner des acides gras. La ou les insaturation(s) seront introduites grâce à l'action de la désaturase d'acides gras codée par le gène *OLE1* (Stukey, 1990).

En ce qui concerne plus particulièrement le phénomène de détoxification, plusieurs mécanismes ont pu être mis en évidence chez la levure. En particulier, la neutralisation des groupements à radicaux libres est dépendante de l'expression du gène *SNQ3* (Moye-Rowley, 1989). Par ailleurs, la levure possède des protéines capables d'expulser dans le milieu externe des xénobiotiques ayant pénétré dans la cellule ou des composés endogènes. Certaines comme *SNQ1* (Kanawaza,1988) ont une spécificité de transport assez stricte, par contre d'autres comme *PDR5* (Leppert, 1990), *SNQ2* (Servos, 1993) et *STE6* (Kuchler, 1989) ont une faible spécificité et peuvent donc transporter un grand nombre de molécules. Différents gènes contrôlant l'expression de ces protéines ont pu être mis en évidence comme les gènes *PDR1* (Meyers, 1992,), *PDR3, PDR4, PDR7, PDR9, PDR11* (Dexter, 1994). Il a notamment été montré que le gène PDRS est régulé par PDR1, PDR3, PDR4, PDR7 et PDR9 (Dexter, 1994). Une autre étude sur la perméabilité cellulaire a mis en évidence le fait qu'un double mutant erg6/erg2 présente une plus grande rigidité membranaire et que ce mutant est perméable au Ni₂₊ (lees 1979; Kleinhans, 1979.) et que ce montant est perméable. D'autres gènes impliqués dans ces mécanismes ont pu être identifiés comme *PDR2* et *PDR6* (Balzi, 1991) mais leurs fonctions demeurent pour l'instant méconnues.

Il faut noter que les protéines codées par les gènes *SNQ2, STE6, PDR5* ressemblent, de par leur composition et de par leur structure aux p-glycoprotéines que l'on retrouve fortement exprimées dans de nombreuses cellules tumorales devenues résistantes à des agents cytotoxiques. Ces protéines sont appelées familièrement pompes MDR (multiple drug résistance).

La maîtrise des différents paramètres agissant au niveau de ce mécanisme de détoxification et, de manière plus générale, contrôlant indirectement ou directement la perméabilité cellulaire, serait particulièrement précieuse sur un plan thérapeutique.

Notamment, il serait particulièrement avantageux de pouvoir disposer d'une levure présentant une perméabilité accrue à des fins de criblage de produits biologiquement actifs ou d'études de métabolismes et/ou de comportements cellulaires. En effet, dans ce type de levures, la concentration intracellulaire d'un composé serait accrue par rapport à des levures normales, pour une même concentration du produit dans le milieu externe. Cette concentration intracellulaire augmentée permettrait une détection plus facile de l'activité du produit dans le cadre du criblage et la découverte de composés actifs (Kirsch 1993, Hara 1993). Elle serait également avantageuse pour obtenir une plus grande transformation d'un substrat lors d'une biocatalyse (Yasubaki 1992) ou pour examiner le métabolisme d'un composé, en utilisant par exemple des levures exprimant des cytochromes p450 humains (Renaud 1990).

De manière inattendue, la Demanderesse a mis en évidence que la combinaison, chez une levure, d'au moins deux mutations au niveau d'au moins deux des gènes précités et contrôlant chacun la perméabilité, à des niveaux différents ou non, affectait positivement la perméabilité de ladite levure. Il s'ensuit un gain accru de perméabilité. Ce gain correspond au moins à celui issu de la superposition des effets respectifs induits, par chacune des mutations, et plus préférentiellement lui est nettement supérieur. On assiste avantageusement à une addition et plus préférentiellement à une amplification des effets respectifs c'est à dire à une synergie.

Plus précisément, la présente invention se rapporte à une levure modifiée dans laquelle au moins deux des gènes contrôlant la perméabilité cellulaire de ladite levure sont modifiés, lesdites modifications ayant un effet additif ou synergique.

Les gènes affectés dans leur fonction, dans le cadre de la présente invention, peuvent contrôler la perméabilité cellulaire à des niveaux identiques ou différents. Toutefois, il peut être avantageux d'altérer l'activité de gènes affectant la perméabilité cellulaire à des niveaux distincts.

Ces gènes modifiés sont choisis parmi les gènes intervenant au niveau de la constitution, la biosynthèse et/ou l'intégrité de la paroi ou de la membrane plasmique et les gènes impliqués dans les mécanismes de détoxification ou de l'export de composés endogènes.

Au sens de la présente invention, on entend par gène modifié, un gène rendu partiellement ou totalement incapable de coder pour sa protéine naturelle. L'incapacité desdits gènes à coder pour leurs protéines naturelles peut se manifester soit par la production d'une protéine inactive en raison de modifications structurales ou conformationnelles, soit par l'absence de production, soit par la production de la protéine naturelle à un niveau atténué ou selon un mode de régulation désiré ou encore par la production d'un mutant ayant un spectre d'activité modifié. Dans ce dernier cas, il peut s'agir d'une modification de type augmentation ou diminution de l'activité spécifique mais également d'un changement au niveau de sa spécificité de substrat ou de produit.

Cette modification des gènes est induite par une ou plusieurs modifications génétiques.

Par modification génétique, on doit entendre plus particulièrement toute suppression, substitution, délétion, insertion ou addition d'une ou plusieurs bases dans le ou les gènes considérés. De telles modifications peuvent être obtenues *in vitro* (sur de l'ADN isolé) ou *in situ,* par exemple, au moyens des techniques du génie génétique, ou encore en exposant lesdits microorganismes à un traitement au moyen d'agents mutagènes. Par agents mutagènes, on peut citer par exemple les agents physiques tels que les rayonnements énergétiques (rayons X, rayon gamma, ultra violet, etc..), ou les agents chimiques capables de réagir avec différents groupements fonctionnels des bases de l'ADN, et par exemple les agents alkylants [éthylméthane sulfonate (EMS), N-méthyl-N-nitro-N-nitrosoguanidine, N-nitroquinoléine-1-oxyde (NQO) etc...], les agents bialkylants, les agents intercalants, etc... Par délétion, on entend toute suppression partielle ou totale du gène considéré. Il peut s'agir notamment d'une partie de la région codant pour lesdites protéines, et/ou de tout ou partie de la région promotrice de la transcription, de la traduction ou encore du transcript.

La ou lesdites modifications génétiques peuvent également être obtenues par disruption génique, par exemple selon le protocole initialement décrit par Rothstein (Rothstein, 1983) ou avantageusement par double recombinaison homologue. Dans ce cas, la séquence codante sera, en partie bu en totalité, préférentiellement perturbée pour permettre le cas échéant, le remplacement, par recombinaison homologue, de la séquence génomique sauvage par une séquence non fonctionnelle, mutante et/ou par un marqueur de sélection.

La ou lesdites modifications génétiques peuvent être localisées dans le gène codant ou en dehors de la région codante, par exemple dans les régions responsables de l'expression et/ou de la régulation transcriptionnelle ou post-transcriptionnelle desdits gènes.

Par ailleurs, certaines altérations telles que des mutations ponctuelles sont par nature capables d'être corrigées ou atténuées par des mécanismes cellulaires, par exemple lors de la réplication de l'ADN précédant la division cellulaire. De telles altérations génétiques ont alors un intérêt limité au niveau industriel puisque les propriétés phénotypiques qui en résultent ne sont pas parfaitement stables. La demanderesse a utilisée un procédé permettant de préparer des levures présentant au moins deux modifications génétiques au niveau de deux gènes respectifs, la ou lesdites modifications étant stables ségrégationellement et/ou non-réversibles. Ce procédé autorise en effet la modification de multiples gènes en utilisant le même marqueur de sélection.

Préférentiellement, les levures selon l'invention présentent plusieurs modifications génétiques stables ségrégationellement. Toujours selon un mode préféré, les modifications génétiques sont non-réversibles.

Bien entendu, l'objet de la présente invention n'est pas limité aux levures comportant uniquement deux gènes inactivés. Les levures modifiées selon l'invention peuvent contenir un nombre nettement supérieur de gènes mutés.

Les levures selon l'invention peuvent être obtenues en modifiant au moins deux gènes choisis parmi les gènes intervenant au niveau de la constitution, la biosynthèse et/ou l'intégrité de la paroi et/ou de la membrane plasmique, et/ou les gènes impliqués dans les mécanismes de détoxification ou d'export de composés endogènes, leurs homologues ou dérivés.

Au sens de la présente invention, on entend par dérivé, toute séquence obtenue par modification et codant pour un produit conservant l'une au moins des propriétés biologiques de la protéine naturelle (effet trophique et/ou différentiateur). Par modification, on doit entendre toute mutation, substitution, délétion, addition ou modification de nature génétique et/ou chimique. Les dérivés au sens de l'invention peuvent également être obtenus par hybridation à partir de banques d'acides nucléiques, en utilisant comme sonde la séquence native ou un fragment de celle-ci.

Parmi les dérivés préférés, on peut citer plus particulièrement les variants naturels, les molécules dans lesquelles un ou plusieurs résidus ont été substitués, les dérivés obtenus par délétion de régions n'intervenant pas ou peu dans l'interaction avec les sites de liaison considérés ou exprimant une activité indésirable, et les dérivés comportant par rapport à la séquence native des résidus supplémentaires.

Par gène homologue, on entend désigner dans le cadre de la présente invention un gène remplissant une fonction identique ou équivalente à celle d'un gène expressément désigné et isolé à partir d'un même ou d'un autre microorganisme.

Plus préférentiellement, les gènes susceptibles d'être modifiés dans le cadre de la présente invention sont sélectionnés parmi:
- les gènes qui interviennent au niveau de la synthèse de la paroi cellulaire ou dans le maintien de son intégrité. A titre représentatif de ces gènes on peut notamment mentionner les gènes *KRE, KTR, MNN, CSD, CHS, OCH, SKN1, PMR1, PMT1, ERD1, VGR1, VGR4, CAL3, SHC1, DHS1, SRBI, PKC1, BCK1, MKK1, MKK2, MPK1, PPZ1, PPZ2, PMI1* et *BGL2,*
- les gènes qui interviennent au niveau de la synthèse de la paroi cellulaire en participant notamment à la synthèse des stérols, constituants des membranes cellulaires, et plus particulièrement les gènes *ERG10, ERG11, ERG13, HMG1, HMG2, ERG12, ERG8, ERG19, ID11, ERG20, ERG9, ERG1, ERG18, ERG7, ERG17, ERG16, ERG24, ERG6, ERG2, ERG3, ERG5* et *ERG4,*
- les gènes impliqués dans les mécanismes de détoxification ou d'export de composés endogènes développés par la cellule tels que les gènes *SNQ1, SNQ2, SNQ3, STE6, PDR1, PDR2, PDR3, PDR4, PDR6, PDR7, PDR9* et *PDR11* et leurs homologues.

Les cellules de levures susceptibles d'être modifiées selon l'invention peuvent être choisies parmi les genres *Kluyveromyces, Saccharomyces, Pichia, Hansenula, Candida, Schizosaccharomyces* et leurs analogues et plus préférentiellement entre les genres *Kluyveromyces, Saccharomyces.* L'espèce préférée de *Saccharomyces* est *S. Cerevisiae.*

Selon un mode particulier de l'invention, les mutations peuvent être effectuées au niveau d'au moins deux gènes contrôlant la perméabilité des levures à des niveaux différents. Par exemple, il peut s'agir d'une levure associant à une inactivation d'un gène impliqué dans les mécanismes de détoxication, une inactivation d'un gène impliqué dans la synthèse des stérols.

A titre illustratif des levures revendiquées on peut notamment citer plus particulièrement les levures dans lesquelles les gènes *PDR1, PDR5, SNQ2, ERG6* ont été modifiés comme dans la souche yCM61, décrite dans les exemples ci-après.

Dans ce cas particulier, on observe une synergie particulièrement avantageuse. Une telle levure possède une perméabilité nettement accrue qui se traduit par une hypersensibilité à des produits cytotoxiques ayant des cibles intracellulaires et des mécanismes d'action différents (voir exemples).

L'invention a également pour objet un procédé de préparation d'une levure génétiquement modifiée et son utilisation. Préférentiellement, le procédé de l'invention consiste à remplacer les gènes chromosomiques considérés par une version modifiée *in vitro.*

La présente invention vise de manière générale toute utilisation d'une levure selon l'invention à des fins de pénétration d'un composé dans ladite levure. Les levures présentant une perméabilité accrue selon l'invention s'avèrent tout particulièrement avantageuses pour la découverte de produits biologiquement actifs dans des cribles microbiologiques (Kirsch 1993, Hara 1993). Par ailleurs, elles sont intéressantes en biocatalyse (Yabusaki 1992) ou en tant que modèle pour l'étude de métabolisme, pour la compréhension du comportement cellulaire et pour les études de toxicologie.

La présente invention sera plus complètement décrite à l'aide des figures et exemples suivants, qui doivent être considérés comme illustratifs et non limitatifs.

### LEGENDE DES FIGURES:

Figure 1: Plasmide pDIS1
Figure 2: Plasmide pDIS3
Figure 3: Plasmide pDIS4
Figure 5: Plasmide pCM2
Figure 6: Plasmide pCM4
Figure 7: Plasmide pCM5
Figure 8: Plasmide pCM6
Figure 9: Plasmide pCM7
Figure 10: Plasmide pCM8
Figure 11: Plasmide pCM12
Figure 12: Plasmide pCM13
Figure 13: Plasmide pCM10
Figure 14: Plasmide pCM11

### TECHNIQUES GENERALES DE CLONAGE.

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc..., sont bien connues de l'homme de métier et sont abondamment décrites dans la littérature [Maniatis T. et coll., "Molecular Cloning, a Laboratory Manual", Cold Spring Harbor Laboratory, Cold Spring Harbor, N.Y., 1982; Ausubel F.M. et coll. (eds), "Current Protocols in Molecular Biology", John Wiley & Sons, New York, 1987].

Les enzymes de restriction ont été fournies par New England Biolabs (Biolabs), Bethesda Research Laboratories (BRL) ou Amersham et sont utilisées selon les recommandations des fournisseurs.

Les plasmides de type pBR322, pUC et les phages de la série M13 sont d'origine commerciale (Bethesda Research Laboratories). Les plasmides de type pIC ont été décrits par Marsh (Marsh, 1984).

Pour les ligatures, les fragments d'ADN sont séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes est effectué par le fragment de Klenow de l'ADN Polymérase I d'*E. Coli* (Biolabs) selon les spécifications du fournisseur. La destruction des extrémités 3' proéminentes est effectuée en présence de l'ADN Polymérase du phage T4 (Biolabs) utilisée selon les recommandations du fabricant.

Les oligodéoxynucléotides sont synthétisés chimiquement selon la méthode des phosphoramidites en utilisant des groupements protecteurs β-cyanoéthyles (Sinha 1984). Après synthèse, les groupements protecteurs sont éliminés par traitement à l'ammoniaque et deux précipitations au butanol permettent de purifier et de concentrer les oligodéoxynucléotides (Sawadogo, 1991). La concentration en ADN est déterminée par mesure de la densité optique à 260 nm.

Les transformations de S cerevisiae avec l'ADN des plasmides d'expression des protéines de la présente invention sont effectuées par toute technique connue de l'homme de l'art, et dont un exemple est donné dans le texte.

Les souches de levures utilisées dans les exemples appartiennent à l'espèce *Saccharomyces cerevisiae.*

Les souches de levures de la présente invention sont cultivées en erlenmeyers à 28°C en milieu riche (YPG: 1% extrait de levure, 1% Bactopeptone, 2% glucose) ou un milieu minimum (YNB: 0,67% Yeast Nitrogen Base (difco) sans amino-acides, 2% glucose) sous agitation constante. Le milieu minimum est supplémenté en base et en acide aminé le cas échéant à raison de 50mg/L.

### EXEMPLE 1

### Préparation des souches possédant ERG4, PDR1 ou ERG4 et PDR1 inactivés.

### 1)Préparation des plasmides permettant d'effectuer de multiples disruptions géniques avec élimination du marqueur de sélection.

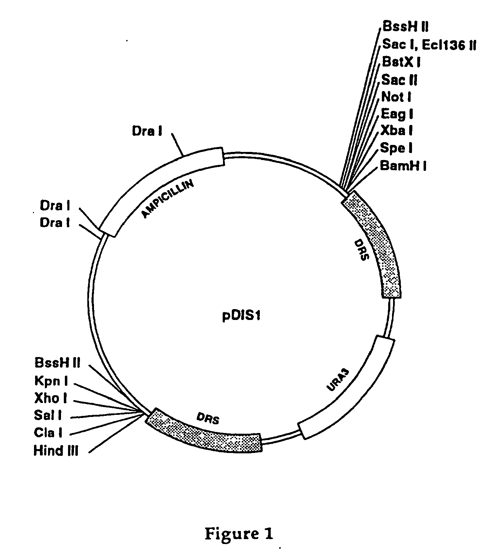

Le plasmide Bluescript II (ks⁻) est digéré par DraI. Les différents fragments de restrictions sont séparés par électrophorèse sur gel d'agarose 0,8%. Le fragment de 692 pb correspondant à une partie du gène AMP^{R} est ensuite électroélué et purifié par une extraction par un mélange phénol-chloroforme. Ce fragment est ligué dans le plasmide Bluescript II (ks⁻) préalablement digéré par Smal. Après transformation d'*E*. *coli* et analyse des plasmides, ne sont retenus que les plasmides possédant le fragment inséré (le plasmide obtenue est nommé pDisIdrs). Le plasmide pDisIdrs est digéré par EcoRV et ligué avec le fragment DraI-DraI de 692pb décrit ci-dessus. Aprés transformation d'*E*. *coli* et analyse des plasmides, ne sont retenus que les plasmides possédant le fragment inséré en répétition directe avec le précédent (obtention du plasmide pDisI2drs). Le plasmide pCXJI (Chen 1987) possédant le gène *URA3* est digéré par EcoRI. Les différents fragments de restriction sont séparés par électrophorèse sur gel d'agarose 0,8%. Le fragment de 1282 pb est ensuite électroélué et purifié par une extraction par un mélange phénol-chloroforme. Ce fragment est ligué dans le plasmide pDisI2drs préalablement digéré par EcoRI. Après transformation d'*E*. *coli* et analyse des plasmides, ne sont retenus que le plasmide possédant le fragment *URA3* EcoRI-EcoRI inséré. Ce plasmide est appelé pDIS1 (cf figure 1).

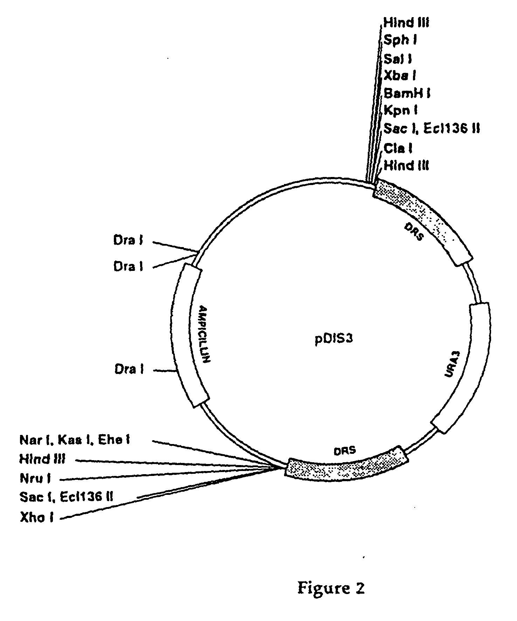

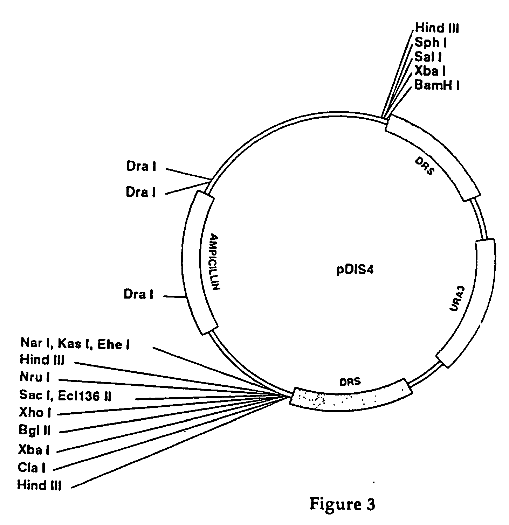

Le plasmide pDIS1 est digéré par ClaI et BamHI. Les différents fragments de restrictions sont séparés par électrophorèse sur gel d'agarose 0,8%. Le fragment de 2702 pb portant le gène *URA3* flanqué des deux séquences en répétition directe est ensuite électroélué et purifié par une extraction par un mélange phénol-chloroforme. Ce fragment est ligué dans le plasmide pIC20H digéré d'une part par ClaI et BamHI et d'autre part par ClaI et BglII. Les nouveaux plasmides ayant ligué ce fragment de 2702 pb, nommé respectivement pDIS3 (cf figure 2) et pDIS4 (cf figure 3), sont obtenus après transformation d'*E*. *coli.*

### 2) Obtention des contructions permettant la disruption des gènes ERG4, PDR1 et ERG4-PDR1.

### a) Construction permettant la disruption du gène ERG4

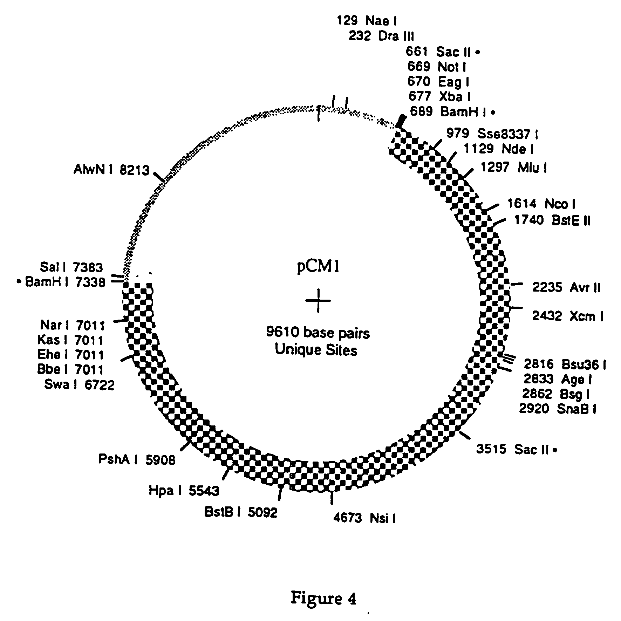

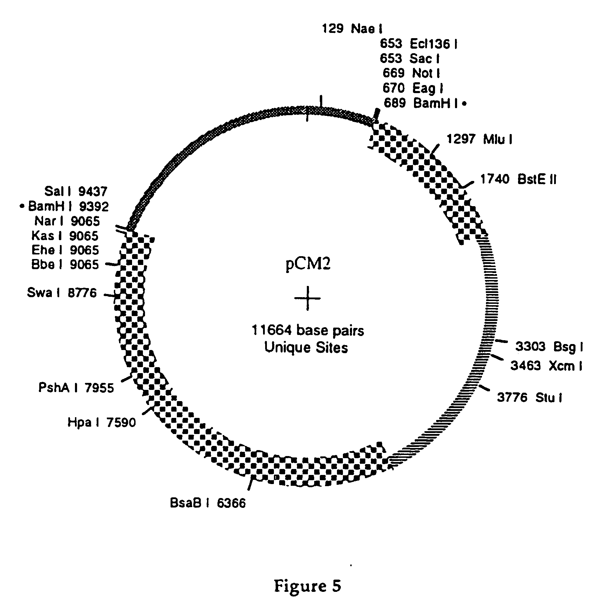

Le fragment BamHI-BamHI de 6670 pb du plasmide pA-B6.5 (Balzi 1987) dont la séquence est déposée dans la banque de données EMBL sous la référence S58126 est ligué dans le plasmide Bluescript II (ks-) préalablement digéré par BamHI. Ce fragment d'ADN comporte entre autre les ORF correspondant aux gènes *ERG4* et *PDR1.* Après transformation d'*E coli,* le plasmide possédant l'insert est retenu et nommé pCM1 (cf figure 4). Le plasmide pDIS4 est digéré par XbaI et Ec1136II. Les différents fragments de restriction sont séparés par électrophorèse sur gel d'agarose 0,8%. Le fragment de 2725 pb est ensuite électroélué et purifié par une extraction par un mélange phénol-chloroforme. Le plasmide pCM1 est digéré par AvrII et SnaBI de manière à éliminer une partie de l'ORF *ERG4* qui sera remplacée par la cassette de disruption. Les différents fragments de restriction sont séparés par électrophorèse sur gel d'agarose 0,8%. Le fragment de 8925 pb est ensuite électroélué et purifié par une extraction par un mélange phénol-chloroforme. Les deux fragments ainsi préparés sont ligués ensemble. Après transformation d*'E*. *coli* le plasmide permettant la disruption du gène *ERG4* est préparé. Ce nouveau plasmide est appelé pCM2 (cf figure 5). Il est finalement digéré par SacI et SalI. Le fragment linéaire ainsi obtenu est électroélué et purifié par une extraction par un mélange phénol chloroforme, en vue de la transformation permettant la disruption du gène *ERG4*.

### b) Construction permettant la disruption des gènes PDR1 et ERG4

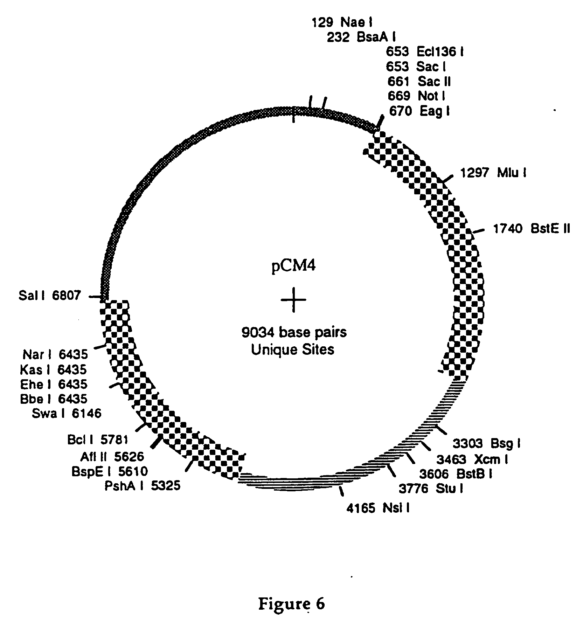

Le plasmide pCM1 est digéré par AvrII et HpaI de manière à éliminer une partie de l'ORF *ERG4* et de l'ORF *PDR1* qui sera remplacée par la cassette de disruption. Les différents fragments de restriction sont séparés par électrophorèse sur gel d'agarose 0,8%. Le fragment de 6302 pb est ensuite électroélué et purifié par une extraction par un mélange phénol-chloroforme. Le plasmide pDIS4 est digéré par XbaI et Ecl136II pour obtenir la cassette de disruption compatible. Les différents fragments de restrictions sont séparés par électrophorèse sur gel d'agarose 0,8%. Le fragment de 2725 pb est ensuite électroélué et purifié par une extraction par un mélange phénol-chloroforme. Les deux fragments ainsi préparés sont ligués ensemble. Après transformation d'*E. coli,* le plasmide nommé pCM4 (cf figure 6) permettant la disruption des gènes *ERG4* et *PDR1* est préparé. Il est finalement digéré par SacI et SalI. Le fragment linéaire ainsi obtenu est électroélué et purifié par une extraction par un mélange phénol chloroforme, en vue de la transformation permettant la disruption des gènes *ERG4* et *PDR1.*

### c) Construction permettant la disruption du gène PDR1

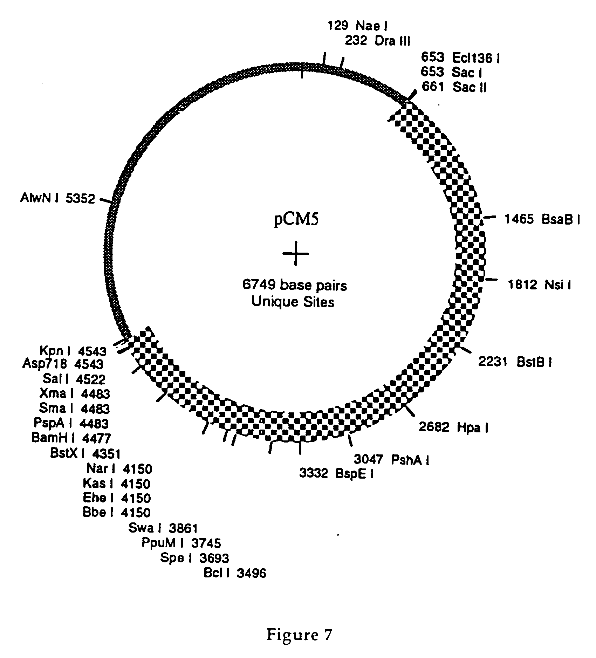

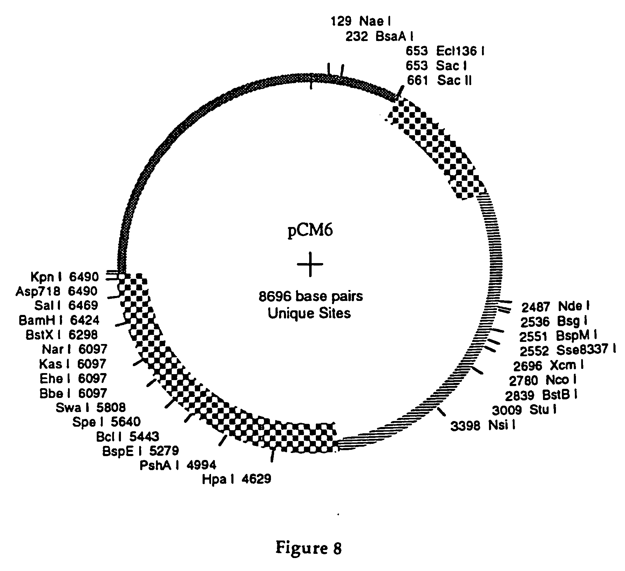

Le plasmide pCM1 est digéré par SacII puis extrait par un mélange phénol chloroforme et enfin religué de manière à éliminer un fragment d'ADN genant pour la construction permettant la disruption du gène *PDR1.* Après transformation d'*E*. *Coli* le plasmide ayant éliminé le fragment de 2854 pb est obtenu et désigné pCM5 (cf figure 7). Ce plasmide est ensuite digéré par BstBI et BsaBI. Les différents fragments de restriction sont séparés par électrophorèse sur gel d'agarose 0,8%. Le fragment de 5983 pb est ensuite électroélué et extrait par un mélange phénol-chloroforme. Le plasmide pDIS3 est digéré par ClaI et Ec1136II. Les différents fragments de restriction sont séparés par électrophorèse sur gel d'agarose 0,8%. Le fragment de 2709 pb est ensuite électroélué et extrait par un mélange phénol-chloroforme. Les deux fragments ainsi préparés sont ligués ensemble. Après transformation d'*E*. *Coli* le plasmide permettant la disruption du gène *PDR1,* nommé pCM6 (cf figure 8) est préparé. Il est finalement digéré par SalI et SacI. Le fragment linéaire ainsi obtenu est électroélué et purifié par une extraction par un mélange phénol chloroforme, en vue de la transformation permettant la disruption du gène *PDR1.*

### 3) Transformation de la levure

La souche de levure utilisé est la souche *S. cerevisiae* 5247 (Marcireau, 1992) de génotype *ura3, trp1, MAT a.* Cette souche est transformée selon la méthode décrite par Gietz. (Gietz, 1992).
Les clones ayant intégré la cassette de disruption sont sélectionnés pour leur prototrophie pour l'uracile. Après avoir vérifié que l'intégration s'est faite au locus (obtention du phénotype attendu) la cassette *URA3* est perdue après repiquage sur milieu complet. Les clones ayant perdu la cassette *URA3* par recombinaison des deux séquences répétées sont sélectionnés sur un milieu contenant 10 mM de 5-fluoroorotate.

### 4) Obtention des souches de levure possédant les gènes PDR1, ERG4 et PDR1-ERG4 modifiés.

La souche 5247 est transformée comme décrit en 3) par les fragments préparés comme décrit en 2a), 2b), 2c). Aprés élimination du marqueur de selection, les souches yCM30 (Δ*pdr1, ura3, trp1, MAT a*), yCM63 (Δ*erg4, ura3, trp1, MAT a*) et yCM65 (Δ*pdr1,* Δ*erg4, ura3, trp1, MAT a*) sont obtenues.

### EXEMPLE 2:

### Evaluation de la perméabilité de la levure obtenue selon l'exemple 1

La perméabilité des levures obtenues en exemple 1 est déterminée par la mesure de la sensibilité à différent produits cytotoxiques. Les différentes levures sont ensemencées à une densité optique de 0,05 à 700nm dans du milieu minimum YNB correctement supplémenté en bases et acides aminés en présence d'une concentration croissante de xénobiotiques. Les cultures sont placées à 28°C et à 200 rpm pendant 48 heures. La densité optique à 700nm est alors mesurée. L'EC₅₀ est alors déterminée comme la concentration en xénobiotique inhibant de moitié la croissance de la levure comparativement à un témoin sans xénobiotique.
Le tableau 1 ci-après rend compte des résultats obtenus.

### EXEMPLE 3:

### Préparation des souches possédant ERG6 et ERG6-PDR1 inactivés.

### 1) Obtention des contructions permettant la disruption des gènes ERG6 et ERG6, PDR1.

### a) construction permettant la disruption du gène ERG6

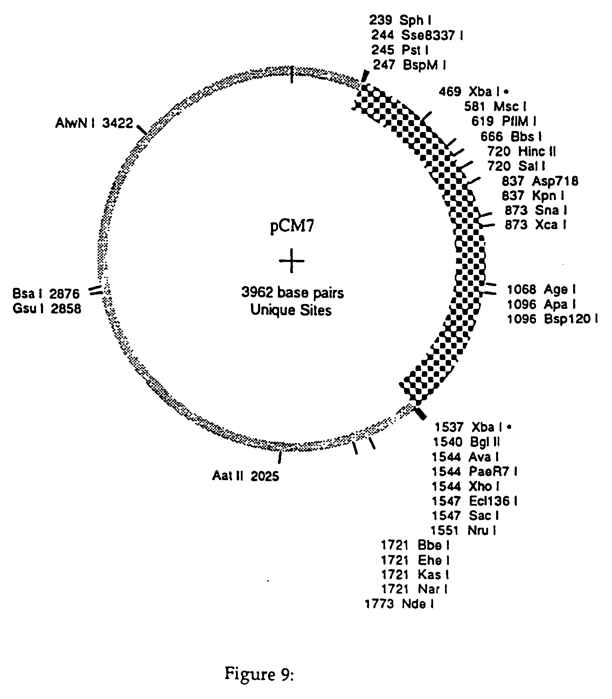

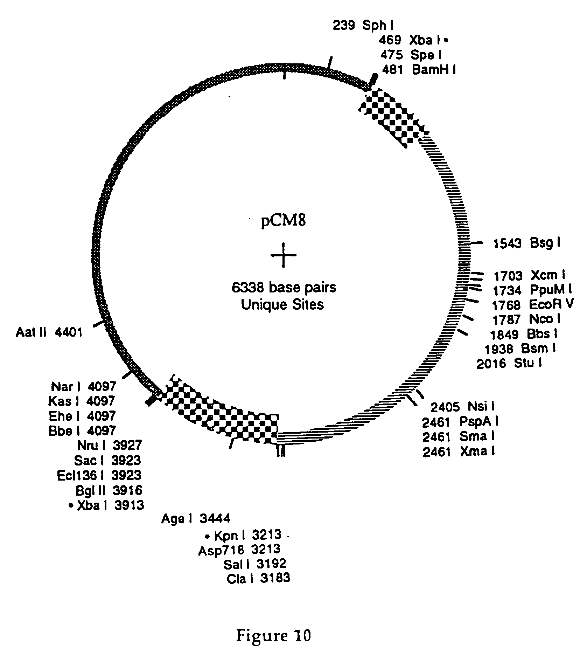

Le gène ERG6 est obtenu par PCR afin d'introduire des sites de restriction adéquats. Les amorces utilisées sont TTA CTT TCG ATT TAA GTT TTA CAT AAT TTA AAA SEQ ID N°1 pour le bord 5' et GGC CTG CTC ATG AAC GTG CTA TCT T SEQ ID N°2 pour le bord 3'. Le programme de température pour la PCR est le suivant: 2 minutes à 91°C puis 30 cycles d'une minute à 91°C puis 2 minutes à 45°C et 3 minutes à 72°C et pour terminer 5 minute à 72°C. Le produit de PCR après électrophorèse sur gel d'agarose 0,8% est électroelué et extrait par un mélange phénol-chloroforme. Puis il est digéré par Dral et XhoI tandis que le pIC20H est digéré par EcoRV et XhoI. Après électrophorèse sur gel d'agarose 0,8% les deux fragments sont électroélués et extraits par un mélange phénol-chloroforme et ligués ensemble. Après transformation d'*E*. *Coli,* le plasmide baptisé pCM7 (cf figure 9) est préparé à partir des colonies obtenues. Ce dernier est digéré par XbaI et KpnI. Les différents fragments de restriction sont séparés par électrophorèse sur gel d'agarose 0,8%. Le fragment de 3594 pb est ensuite électroélué et extrait par un mélange phénol-chloroforme. Le plasmide pDIS1 est digéré par XbaI et Kpnl. Les différents fragments de restriction sont séparés par électrophorèse sur gel d'agarose 0,8%. Le fragment de 2744 pb est ensuite électroélué et extrait par un mélange phénol-chloroforme. Les deux fragments ainsi préparés sont ligués ensemble. Aprés transformation d'*E*. *Coli* le plasmide pCM8 est obtenu (cf figure 10). Pour la disruption d'*ERG6* il est finalement digéré par BamHI et BglII. Le fragment linéaire ainsi obtenu est électroélué et purifié par une extraction par un mélange phénol chloroforme, en vue de la transformation permettant la disruption du gène *ERG6*

### 2) Transformation de la levure

Une levure est transformée selon le protocole décrit en exemple 1.3).

### 3) Obtention des souches délétées pour ERG6 et ERG6,PDR1

La souche 5247 est transformée par le fragment préparé comme décrit en la. Après élimination du marqueur de sélection la souche yCM55 (Δ*erg6, urα3, trp1, MAT a*) est obtenue.
La souche yCM30 est transformée par le fragment préparé comme décrit en la. Après élimination du marqueur de sélection la souche yCM57 (Δ*pdr1*, *Δerg6, ura3, trp1, MAT a*) est obtenue.

### EXEMPLE 4:

### Evaluation de la perméabilité des souches de levure yCM30, yCM55, yCM57.

Cette évaluation a été réalisée selon le protocole décrit en exemple 2. Les résultats correspondants figurent en tableau II.

### EXEMPLE 5:

### Préparation des souches de levures possédant PDR5 et SNQ2 inactivés

### 1) Obtention des contructions permettant la disruption des gènes PDR5 et SNQ2. a) disruption du gène PDR5

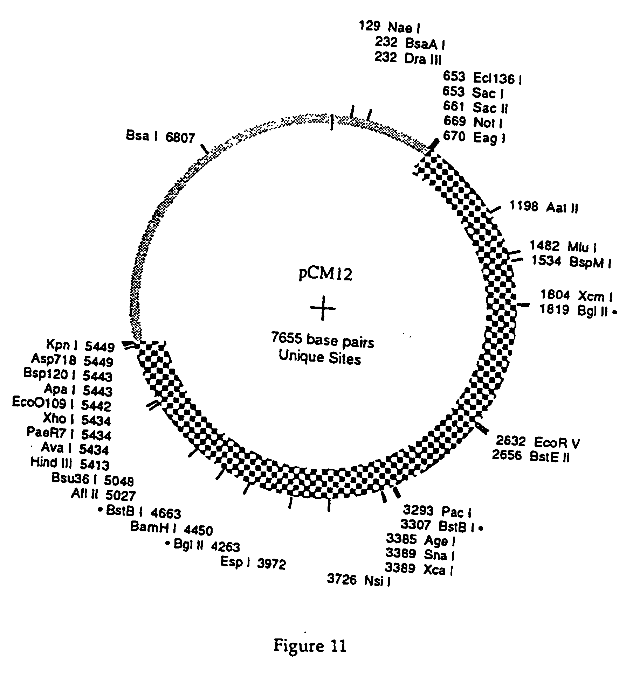

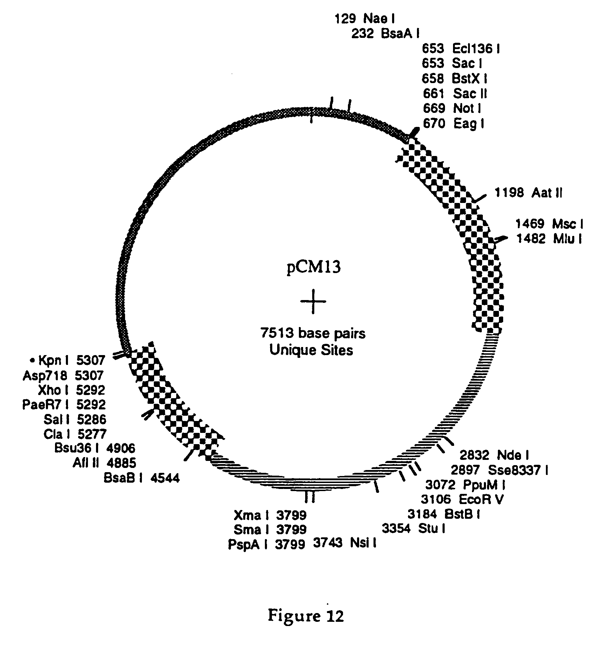

Le fragment d'ADN AvrII-HindIII (comportant une partie de l'ORF *PDR5*) de 4736 pb du plasmide pDR3.3 (Leppert 90) dont la séquence est déposée dans la banque de données EMBL sous la référence L19922 est ligué dans le plasmide bluescript II (ks) préalablement digéré par HindIII et XbaI. Après transformation d'*E.Coli,* le plasmide baptisé pCM12 possédant l'insert est retenu (cf figure 11). Ce plasmide est ensuite digéré par BstBI et BglII. Les différents fragments de restriction sont séparés par électrophorèse sur gel d'agarose 0,8%. Le fragment de 4811 pb est ensuite électroélué et extrait par un mélange phénol-chloroforme. Le plasmide pDIS4 est digéré par ClaI et BamHI. Les différents fragments de restriction sont séparés par électrophorèse sur gel d'agarose 0,8%. Le fragment de 2702 pb est ensuite électroélué et extrait par un mélange phénol-chloroforme. Les deux fragments ainsi préparés sont ligués ensemble. Après transformation d'*E*. *Coli* le plasmide nommé pCM13, permettant la disruption de *PDR5,* est préparé (cf figure 12). Il est finalement digéré par ClaI et SacI. Le fragment linéaire ainsi obtenu est électroélué et purifié par une extraction par un mélange phénol chloroforme, en vue de la transformation permettant la disruption du gène *PDR5.*

### b) disruption du gène SNQ2

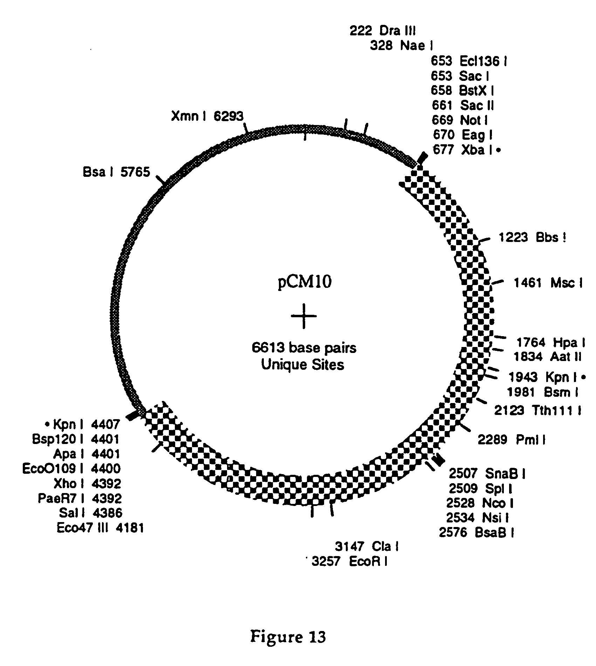

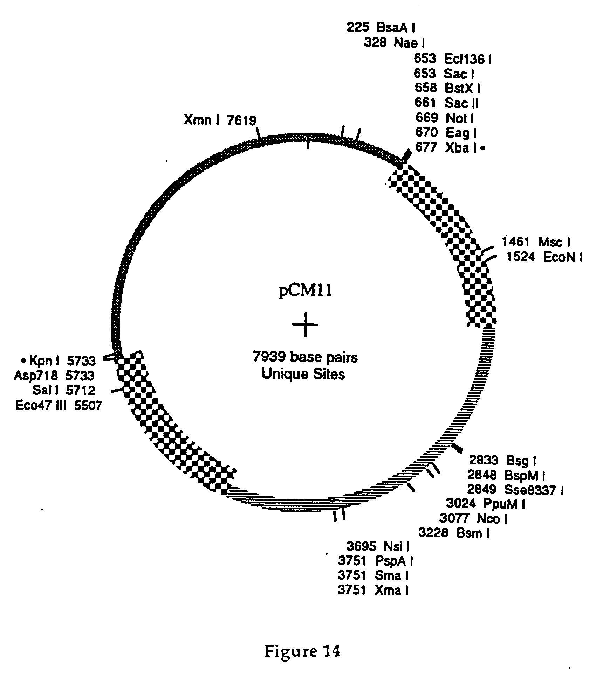

Le fragment d'ADN NarI-BgIII de 3688 pb (comportant une partie de l'ORF *SNQ2*) du plasmide pEH5222 (Servos 1993) dont la séquence est déposée dans la banque de données EMBL sous la référence X66732 est ligué dans le plasmide Bluescript II (ks-) préalablement digéré par BamHI et ClaI. Après transformation d'*E*. *coli,* le plasmide baptisé pCM10 possédant l'insert est retenu (cf figure 13). Ce plasmide est ensuite digéré par ClaI et HpaI de manière à éliminer une partie de l'ORF *SNQ2* qui sera remplacée par la cassette de disruption. Les différents fragments de restriction sont séparés par électrophorèse sur gel d'agarose 0,8%. Le fragment de 5230 pb est ensuite électroélué et purifié par une extraction par un mélange phénol-chloroforme. Le plasmide pDIS3 est digéré par ClaI et Ecl136II pour obtenir la cassette de disruption compatible.. Les différents fragments de restriction sont séparés par électrophorèse sur gel d'agarose 0,8%. Le fragment de 2709 pb est ensuite électroélué et purifié par une extraction par un mélange phénol-chloroforme. Les deux fragments ainsi préparés sont ligués ensemble. Après transformation d'*E*. *coli* le plasmide nommé pCM11 (cf figure 14) permettant la disruption de *SNQ2* est préparé. Il est finalement digéré par XbaI et SalI. Le fragment linéaire ainsi obtenu est électroélué et purifié par une extraction par un mélange phénol chloroforme, en vue de la transformation permettant la disruption du gène *SNQ2.*

### 2) Obtention des souches délétées pour PDR5et SN02

La souche 5247 est transformée par le fragment préparé comme décrit en 1a et 1b. Après élimination du marqueur de sélection la souche yCM33 (Δ*pdr5, ura3, trp1*, *MAT a*) et la souche yCM67 (Δ*snq2, ura3, trp1, MAT a*) sont obtenues.

### 3) Obtention des souches délétées pour PDR1, PDR5, SNQ2, et ERG6

La souche yCM30 est transformée par le fragment préparé comme décrit dans l'exemple 5 en 1a. Après élimination du marqueur de sélection la souche yCM45 (Δ*pdr1, Δpdr5, ura3, trp1, MAT a*) est obtenue. La souche yCM45 est transformée par le fragment préparé comme décrit dans l'exemple 5 en 1b. Après élimination du marqueur de sélection la souche yCM51 (Δ*pdr1, Δpdr5, Δsnq2, ura3, trp1, MAT a*) est obtenue. La souche yCM51 est transformée par le fragment préparé comme décrit dans l'exemple 1 en 1a. Après élimination du marqueur de sélection la souche yCM61 (Δ*pdr1, Δpdr5, Δsnq2, Δerg6, ura₃, trp1, MAT a*) est obtenue.

### EXEMPLE 6:

### Evaluation de la perméabilité de la souche de levure obtenue en exemple 5

La perméabilité des souches de levures 5247 et yCM61 est déterminée par la mesure de la sensibilité à différent produits cytotoxiques. Les différentes levures sont ensemencées à une densité optique de 0,05 à 700nm dans du milieu minimum YNB correctement supplémenté en bases et acides aminés en présence d'une concentration croissante de xénobiotiques. Les cultures sont placées à 28°C et à 200 rpm pendant 48 heures. La densité optique à 700nm est alors mesurée. L'EC₅₀ est déterminée comme la concentration de xenobiotique inhibant de moitié la croissance de la levure comparativement à un témoin sans xénobiotique.

Le tableau III ci-après rend compte des résultats obtenus.

**TABLEAU 1**

| | **DOXORUBICINE** | | **ACTYNOMICINE D** | | **CYCLOHEXIMIDINE** | |
|---|---|---|---|---|---|---|
| SOUCHE | **EC₅₀ (µm)** | **RAPPORT** | **EC₅₀ (µm)** | **RAPPORT** | **EC₅₀ (µm)** | **RAPPORT** |
| 5247 (wt) | 40,5 | 1 | 23,9 | 1 | 53 | 1 |
| yCM63 (Δ*erg*4) | 9,2 | 4,4 | 6,4 | 3,7 | - | - |
| yCM30 (Δ*pdr1*) | 18,4 | 2,2 | 12,7 | 1,9 | 2,5 | 21,2 |
| yCM65 (Δ*pdr1,* Δ*erg4*) | 5,15 | 7,8 | 5,8 | 4,1 | 0,5 | 106 |

**TABLEAU II**

| | **DOXORUBICINE** | | **ACTYNOMICINE D** | | **CYCLOHEXIMIDINE** | |
|---|---|---|---|---|---|---|
| souche | **EC₅₀ (µM)** | **RAPPORT** | **EC₅₀ (µM)** | **RAPPORT** | **EC₅₀ (µM)** | **RAPPORT** |
| 5247 (wt) | 40,5 | 1 | 23,9 | 1 | 53 | 1 |
| yCM55 (Δ*erg6*) | 12 | 3,4 | 5,6 | 4,3 | 2,3 | 23 |
| yCM30 (Δ*pdr1*) | 18,4 | 2,2 | 12,7 | 1,9 | 2,5 | 21,2 |
| yCM57 (Δ*pdr1,* Δ*erg6*) | 1,7 | 23,8 | 0,75 | 31,9 | 0,2 | 265 |

**TABLEAU III**

| **COMPOSE** | 5247 (wt) EC₅₀(µM) | yCM61 (Δ*pdr1,* Δ*pdr5,* Δ*snq2,* Δ*erg6)* EC₅₀(µM) | **RAPPORT** |
|---|---|---|---|
| **Novobiocine** | 245 | 9 | 27 |
| **Lovastatine** | 143 | 3.8 | 37 |
| **Sulfométhtiron** | 6.9 | 0.13<< | >>50 |
| **Camptothécine** | >>574 | 5,74 | >>100 |
| **Cyclohéximide** | 71 | 0.142 | 500 |

### REFERENCES BIBLIOGRAPHIQUES

- Al-Fell et Coll., Proc. Natl. Acad. Sci. USA 1992, 89, 4534,
- Anderson et Coll., J. Biol Chem. 1989, 264, 19169,
- Ashman et Coll., Lipids 1991, 26, 628,
- Arthington et Coll., Gene 1991, 102, 39,
- Ballou 1982, The molecular biology of the yeast Saccharomyces, metabolism and gene expression, eds Cold Spring Harbor Laboratory, 335,
- Balzi et Coll., Biochim, Biophys. Acta 1991, 1073, 241,
- Basson et Coll., Proc. Natl. Acad. Sci. USA 1986, 53,5563,
- Boone et coll., J. Cell. Biol. 1990, 110, 1833;
- Brown et Coll., Genetics 1993, 133, 837,
- Chen et coll., Curr. Genet. 1987, 12, 185
- Dean-Johnson et Coll., J. Biol. Chem. 1989, 264, 1274,
- Dequin et Coll., Cur. genet. 1988, 13,471,
- Dexter et Coll., Genetics 1994, 136, 505,
- Gietz et coll., Nucl. Acids Res. 1992, 20, 1425,
- Fegueur et Coll., Cur. Genet 1991, 20, 365,
- Hara et coll., Proc. Natl. Acad. Sci. USA 1993, 90, 2281
- Hardwick et Coll., Yeast 1994, 10, 265,
- Hill et Coll., Genetics 1992, 130, 273,
- Hjelmstad et Coll., J. Biol. Chem. 1991, 266, 4357,
- Hoegenauer et Coll., Gene 1991, 107, 155,
- Hosaka et Coll., J. Biol. Chem. 1989, 264, 2053,
- Hutchins et coll., J. Bacteriol. 1983, 154, 161,
- Kalb et Coll, DNA 1987, 6, 529,
- Kanawaza et Coll., Mol. Cell. Biol. 1988, 8, 664,
- Kanik et Coll., Mol. Cell. Biol. 1990, 10, 898,
- Karst et Coll., Molec. Gen. Genet 1977, 154, 269,
- Kiyono et Coll., J. Biochem 1987, 102, 1089,
- Klis, Yeast 1994, 10, 851,
- Kodaki et Coll., J. Biol. Chem. 1987, 262, 15428),
- Kodaki et Coll., J. Biochem 1991, 109, 276,
- Kuchler et Coll., EMBO J. 1989, 8, 3973,
- Lai et Coll., Gene 1994, 140, 41,
- Lee et coll., biosciences biotechnology and biochemistry, 1994, 58,391,
- Leppert et Coll., Genetics 1990, 125, 30,
- Lussier et Coll., Yeast 1993, 9; 1057,
- Marcireau et Coll., Cur. Genet. 1992, 22, 267,
- Marsh et coll. Gene 32 (1984) 481,
- Meader et Coll., Mol Cell. Biol. 1990, 10, 3013;
- Meyers et Coll., Curr. Genet. 1992, 21, 431,
- Mohamed et Coll., J. Biol. Chem. 1990, 263, 12315,
- Molzhan et Coll., J. Gen. Microbiol 1972, 72, 339,
- Mons, thèse de doctorat,université de Poitiers 1984,
- Moye-Rowley et Coll., Genes et development 1989, 3, 283,
- Nikawa et Coll., J. Biol. Chem. 1987, 262, 4876,
- Oulmouden et Coll., Gene 88,253,
- Renaud et coll., Eur. J. Biochem., 1990, 194, 889
- Roemer et Coll., Proc. Natl. Acad.Sci. USA 1991, 88, 11295,
- Rothstein Meth. Enzymol. 101 (1983) 202,
- Sawadogo et Van Dyke, Nucleic Acids Res. 19 (1991) 674,
- Schwezer et Coll., Molec. Gen. Genet 1986, 203, 481,
- Servos et Coll., Mol. Gen. Genet. 1993, 236, 214,
- Servouse et Coll., Biochem. Biophys. Res. Commun. 1984, 123, 424,
- Shimizu et coll., Mol. Gen. Genet. 1994, 242, 641,
- Sinha et coll., Nucleic Acids Res. 12 (1984) 4539,
- Stateva et coll, Mol. Cell. Biol. 1991, 11, 4235,
- Stukey et Coll., J. Biol. Chem. 1990, 265, 20144,
- Tsay et Coll., Mol. Cell. Biol. 1991, 11, 620,
- Tsukagoshi et Coll., Eur. J. Biochem 1987 169, 477.
- Yabusaki, The international review of pharmaceutical technology research and development, 1992, 163.

## Revendications

1. Levure modifiée ayant une perméabilité cellulaire accrue **caractérisée en ce que** au moins deux gènes contrôlant à des niveaux différents, la perméabilité cellulaire choisis parmi :
(a) les gènes intervenant au niveau de la synthèses des stérols, leurs dérivés et homologues,
(b) les gènes intervenant au niveau des mécanismes de détoxification ou de l'export de composés endogènes, leurs dérivés et homologues, sont inactivés.

2. Levure modifiée selon la revendication 1 **caractérisée en ce que** les gènes interviennent au niveau sont choisis parmi les gènes *ERG10, ERG11, ERG13, HMG1, HMG2, ERG12, ERG8, ERG19, IDI1, ERG20, ERG9, ERG1, ERG18, ERG7, ERG17, ERG18, ERG24, ERG6, ERG2, ERG3, ERG5, ERG4* et tout gène homologue.

3. Levure modifiée selon l'une des revendications 1 à 2, **caractérisée en ce que** les gènes intervenant au niveau des mécanismes de détoxification ou d'export de composés endogènes sont choisis parmi les gènes *SNQ1, SNQ2, SNQ3, S7E6, PDR1, PDR2, PDR3, PDR4, PDR6, PDR7, PDR9* et *PDR1* 1 et tout gène homologue.

4. Levure modifiée selon rune des revendications 1 à 3 **caractérisée en ce que** les gènes sont inactivés par une ou plusieurs modifications génétiques, de manière à être partiellement ou totalement incapables de coder pour les protéines naturelles ou actives.

5. Levure modifiée selon l'une des revendications 1 à 4, **caractérisée en ce que** les inactivations sont des mutations ponctuelles ou multiples, substitutions, délétions, additions et/ou modifications de nature génétique et/ou chimique.

6. Levure modifiée selon l'une des revendications 1 à 5, **caractérisée en ce que** les gènes inactivés codent pour des protéines moins ou non fonctionnelles ou pour des mutants ayant un spectre d'activité modifié.

7. Levure modifiée selon l'une des revendications précédentes **caractérisée en ce qu'**elle appartient aux genres *Saccharomyces, Kluyveromyces, Pichia, Hansenula, Candida* ou *Schyzosaccharomyces.*

8. Levure modifiée selon l'une des revendications précédentes, **caractérisée en ce qu'**il s'agit d'une levure S *cerevisiae* dans laquelle les gènes *PDR1, PDR5, SNQ2, ERG6* sont modifiés.

9. Procédé de préparation d'une levure modifiée selon l'une des revendications 1 à 8, **caractérisé en ce que** l'on remplace tout ou partie desdits gènes chromosomiques considérés par une version modifiée in vitro.

10. Utilisation d'une levure modifiée selon l'une des revendications de 1 à 8 en vu de la pénétration d'un composé dans ladite levure.

11. Utilisation d'une levure modifiée selon l'une des revendications de 1 à 8 pour la criblage de composés biologiquement actifs.

12. Utilisation d'une levure modifiée selon l'une des revendications de 1 à 8 en biocatalyse.

13. Utilisation d'une levure modifiée selon l'une des revendications de 1 à 8 à titre de modèle de métabolismes et/ou d'études toxicologiques.

## Claims

1. Modified yeast having increased cellular permeability, **characterized in that** at least two genes controlling, at different levels, cellular permeability, chosen from:
(a) the genes which play a role in the synthesis of sterols, their derivatives and their homologues,
(b) the genes which play a role in the mechanisms of detoxification or of export of endogenous compounds, their derivatives and homologues,
are inactivated.

2. Modified yeast according to Claim 1, **characterized in that** the genes which play a role in the synthesis of sterols are chosen from the genes *ERG10, ERG11, ERG13, HMG1, HMG2, ERG12, ERG8, ERG19, ID11, ERG20, ERG9, ERG1, ERG18, ERG7, ERG17, ERG16, ERG24, ERG6, ERG2, ERG3, ERG5, ERG4* and any homologous gene.

3. Modified yeast according to either of Claims 1 and 2, **characterized in that** the genes which play a role in the mechanisms of detoxification or of export of endogenous compounds are chosen from the genes *SNQ1, SNQ2, SNQ3, STE6, PDR1, PDR2, PDR3, PDR4, PDR6, PDR7, PDR9* and *PDR11* and any homologous gene.

4. Modified yeast according to one of Claims 1 to 3, **characterized in that** the genes are inactivated by one or more genetic modifications, so as to be partially or totally incapable of encoding the natural or active proteins.

5. Modified yeast according to one of Claims 1 to 4, **characterized in that** the inactivations are point or multiple mutations, substitutions, deletions, additions and/or modifications of a genetic and/or chemical nature.

6. Modified yeast according to one of Claims 1 to 5, **characterized in that** the inactivated genes encode proteins which are less functional or nonfunctional or encode mutants having a modified activity spectrum.

7. Modified yeast according to one of Claims 1 to 6, **characterized in that** it belongs to the genera *Saccharomyces, Kluyveromyces, Pichia, Hansenula, Candida* or *Schyzosaccharomyces.*

8. Modified yeast according to one of Claims 1 to 7, **characterized in that** it is a yeast *S. cerevisiae* in which the genes *PDR1, PDR5, SNQ2, EGR6* are modified.

9. Process for the preparation of a modified yeast according to one of Claims 1 to 8, **characterized in that** all or part of the said chromosomal genes considered are replaced by a modified version in vitro.

10. Use of a modified yeast according to one of Claims 1 to 8 for the penetration of a compound into the said yeast.

11. Use of a modified yeast according to one of Claims 1 to 8, for screening biologically active compounds.

12. Use of a modified yeast according to one of Claims 1 to 8, in biocatalysis.

13. Use of a modified yeast according to one of Claims 1 to 8, as model for metabolisms and/or toxicological studies.

## Patentansprüche

1. Modifizierte Hefe mit erhöhter Zellpermeabilität, **dadurch gekennzeichnet, daß** mindestens zwei Gene, die in unterschiedlicher Hinsicht die Zellpermeabilität kontrollieren, und zwar aus der Reihe der
(a) Gene, die in die Synthese von Sterolen, ihren Derivaten und ihren Homologen eingreifen,
(b) Gene, die in Entgiftungsmechanismen oder Mechanismen für den Export von endogenen Verbindungen, ihren Derivaten und Homologen eingreifen,
inaktiviert werden.

2. Modifizierte Hefe nach Anspruch 1, **dadurch gekennzeichnet, daß** die Gene, die in die Sterolsynthese eingreifen aus der Reihe der Gene *ERG10, ERG11, ERG13, HMG1, HMG2, ERG12, ERG8, ERG19, IDI1, ERG20, ERG9, ERG1, ERG18, ERG7, ERG17, ERG16, ERG24, ERG6, ERG2, ERG3, ERG5, ERG4* und allen homologen Genen stammen.

3. Modifizierte Hefe nach einem der Ansprüche 1 oder 2, **dadurch gekennzeichnet, daß** die Gene, die in Entgiftungsmechanismen oder Mechanismen des Exports von endogenen Verbindungen eingreifen, aus der Reihe der Gene *SNQ1, SNQ2, SNQ3, STE6, PDR1, PDR2, PDR3, PDR4, PDR6, PDR7, PDR9* und *PDR11* und allen homologen Genen stammen.

4. Modifizierte Hefe nach einem der Ansprüche 1 bis 3, **dadurch gekennzeichnet, daß** die Gene durch eine oder mehrere genetische Modifikationen inaktiviert werden, und zwar so, daß sie ganz oder teilweise unfähig sind, für die natürlichen oder aktiven Proteine zu codieren.

5. Modifizierte Hefe nach einem der Ansprüche 1 bis 4, **dadurch gekennzeichnet, daß** es sich bei den Inaktivierungen um Punktmutationen oder multiple Mutationen, um Substitutionen, Deletionen, Additionen und/oder Modifikationen genetischer und/oder chemischer Art handelt.

6. Modifizierte Hefe nach einem der Ansprüche 1 bis 5, **dadurch gekennzeichnet, daß** die inaktivierten Gene für weniger funktionsfähige oder nicht funktionsfähige Proteine oder für Mutanten mit einem modifizierten Aktivitätsspektrum codieren.

7. Modifizierte Hefe nach einem der Ansprüche 1 bis 6, **dadurch gekennzeichnet, daß** sie zu den Gattungen *Saccharomyces, Kluyveromyces, Pichia, Hansenula, Candida* oder *Schyzosaccharomyces* gehört.

8. Modifizierte Hefe nach einem der Ansprüche 1 bis 7, **dadurch gekennzeichnet, daß** es sich um eine Hefe S. *cerevisiae,* in der die Gene *PDR1, PDR5, SNQ2, EGR6* modifiziert sind, handelt.

9. Verfahren zum Herstellen einer modifizierten Hefe nach einem der Ansprüche 1 bis 8, **dadurch gekennzeichnet, daß** man alle oder einen Teil der in Betracht gezogenen chromosomalen Gene durch eine in vitro modifizierte Version ersetzt.

10. Verwendung einer modifizierten Hefe nach einem der Ansprüche 1 bis 8 für das Eindringen einer Verbindung in diese Hefe.

11. Verwendung einer modifizierten Hefe nach einem der Ansprüche 1 bis 8 für das Screenen von biologisch aktiven Verbindungen.

12. Verwendung einer modifizierten Hefe nach einem der Ansprüche 1 bis 8 für die Biokatalyse.

13. Verwendung einer modifizierten Hefe nach einem der Ansprüche 1 bis 8 als Modell für Metabolismen und/oder Toxikologieuntersuchungen.
